(19) Europäisches Patentamt
European Patent Office
Office européen des brevets

(11) Veröffentlichungsnummer : **0 022 157**
**B1**

(12) # EUROPÄISCHE PATENTSCHRIFT

(45) Veröffentlichungstag der Patentschrift : **23.12.81**

(21) Anmeldenummer : **80102704.6**

(22) Anmeldetag : **16.05.80**

(51) Int. Cl.³ : **C 07 C 59/76**, C 07 C 51/373, C 07 C 69/738, C 07 C 67/343// C07C59/347, C07C55/02, C07C69/716, C07C69/34

(54) **3-Methyl-5-keto-alpha,omega-alken-disäuren und deren Ester, Verfahren zu ihrer Herstellung und ihre Verwendung zur Herstellung makrocyclischer beta-Methyl-ketone.**

(30) Priorität : **26.05.79 DE 2921430**

(43) Veröffentlichungstag der Anmeldung : **14.01.81 (Patentblatt 81/02)**

(45) Bekanntmachung des Hinweises auf die Patenterteilung : **23.12.81 Patentblatt 81/51**

(84) Benannte Vertragsstaaten : **CH DE FR GB IT LI NL**

(56) Entgegenhaltungen :
**DE - A - 2 348 536**
**DE - B - 1 005 055**

**TETRAHEDRON, Band 20, Nr. 10, Oktober 1964 OXFORD (GB)**
**V.R. MAMDAPUR et al. : « Macrocyclic musk compounds-XI. Synthesis of optically active muscone », Seiten 2601-2604**

(73) Patentinhaber : **HAARMANN & REIMER GMBH**
**Postfach 138**
**D-3450 Holzminden (DE)**

(72) Erfinder : **Bauer, Kurt, Dr.**
**Corveyblick 41**
**D-3450 Holzminden (DE)**
Erfinder : **Hagena, Detlef, Dr.**
**Kleiner Bruch 3**
**D-3470 Hoexter 1 (DE)**

(74) Vertreter : **Dill, Erwin, Dr. et al**
**c/o BAYER AG Zentralbereich Patente Marken und Lizenzen Bayerwerk**
**D-5090 Leverkusen 1 (DE)**

**EP 0 022 157 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# 3-Methyl-5-keto-alpha,omega-alken-disäuren und deven Ester, Verfahren zu ihrer Herstellung und ihre Verwendung zur Herstellung makrocyclischer beta-Methyl-ketone

Die Erfindung betrifft 3-Methyl-5-keto-α,ω-alkendisäuren und deren Ester der allgemeinen Formel

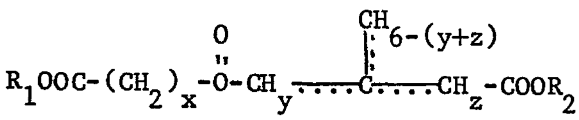

(I)

in der

sich die Doppelbindung in einer durch die gestrichelten Linien gezeigten Stellungen befindet,

$R_1$ und $R_2$ unabhängig voneinander für ein Wasserstoffatom oder eine $C_1$-$C_4$-Alkylgruppe stehen,

x eine ganze Zahl von 9 bis 11 bedeutet und

y und z unabhängig voneinander für 1 oder 2 stehen, mit der Maßgabe, daß y und z nicht gleichzeitig 1 sein dürfen.

Die Erfindung betrifft weiterhin ein Verfahren zur Herstellung der Verbindungen der allgemeinen Formel (I). Das Verfahren ist dadurch gekennzeichnet, daß man ein α,ω-Alkandisäure-Derivat der allgemeinen Formel

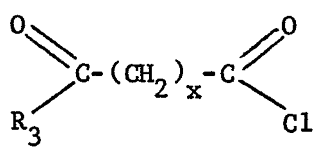

(II)

in der

x die unter Formel (I) angegebene Bedeutung hat und

$R_3$ für Chlor oder eine $C_1$-$C_4$-Alkoxygruppe steht

mit einem Dimethylacrylsäurederivat der allgemeinen Formel

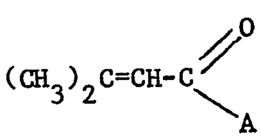

(III)

in der

A für Chlor, einen $C_1$-$C_4$-Alkoxy- oder den β,β-Dimethylacryloyloxi-Rest steht

in Gegenwart einer Lewis-Säure umsetzt.

Außerdem betrifft die Erfindung die Verwendung der erfindungsgemäßen 3-Methyl-5-keto-α,ω-alkendisäuren und Ester der Formel (I) zur Herstellung makrocyclischer β-Methylketone.

Als Vertreter der erfindungsgemäßen Verbindungen der Formel (I) seien beispielsweise genannt:

3-Methyl-5-keto-pentadecen-2-disäure, 3-Methyl-5-keto-pentadecen-3-disäure, 3-Methylen-5-keto-pentadecan-disäure; 3-Methyl-5-keto-hexadecen-2-disäure, 3-Methyl-5-keto-hexadecen-3-disäure, 3-Methylen-5-keto-hexadecan-disäure; 3-Methyl-5-keto-heptadecen-2-disäure, 3-Methyl-5-keto-heptadecen-3-disäure, 3-Methylen-5-ketoheptadecan-disäure; und die Monomethylester, die Dimethylester, die Monoethylester und die Diethylester der genannten Säuren.

Als Vertreter der Verbindungen der Formel (II) seien beispielsweise die Dichloride der α,ω-Undecan-, α,ω-Dodecan, und α,ω-Tridecandisäure, vorzugsweise aber die Monoalkylesterchloride, insbesondere die Monomethyl- und Monoethylester-Chloride der α,ω-Undecan- α-ω-Dodecan- und α-ω-Tridecandisäure genannt.

Als Vertreter der Verbindungen der Formel (III) seien vorzugsweise die $C_1$-$C_4$-Alkylester der Dimethylacrylsäure, wie der Methyl-, Ethyl-, Propyl-, Isopropyl-, n-Butyl- und Isobutylester genannt. Besonders haben sich der Methyl- und der Ethylester bewährt.

Die Verbindungen der Formel (II) sind aus den zugrundeliegenden Säuren nach bekannten Verfahren erhältlich (siehe z.B. J. Am. Chem. Soc. *72*, 5139 (1950)).

Zur Herstellung der erfindungsgemäßen Verbindungen der Formel (I) werden die Verbindungen der Formel (II) mit den Verbindungen der Formel (III) im Molverhältnis 1: 0,9 bis 1,2, vorzugsweise 1: 1 bis 1,1 eingesetzt.

Als Lewis-Säuren können Aluminiumchlorid, Aluminiumbromid, Eisen-(III)-chlorid, Antimonpentachlorid, Zinntetrachlorid, Bortrifluorid und andere als Lewis-Säuren bekannte Verbindungen eingesetzt werden. Bevorzugt werden Aluminiumchlorid und Zinntetrachlorid verwendet. Die Lewis-Säuren werden in Mengen von 1 bis 5 Mol, vorzugsweise 2 bis 3 Mol je Mol Säurehalogenid angewendet.

Die erfindungsgemäße Umsetzung der Verbindungen (II) mit den Verbindungen (III) kann mit oder ohne Lösungsmittel durchgeführt werden. Die Verwendung eines Lösungsmittels ist jedoch vorteilhaft wegen der besseren Temparaturkontrolle und der besseren Homogenisierung der Reaktionsmischung. Als geeignete Lösungsmittel haben sich chlorierte Kohlenwasserstoffe wie Methylenchlorid, Ethylenchlorid, Tetrachlorkohlenstoff, Tetrachlorethylen und Dichlorbenzol, ferner Schwefelkohlenstoff, Nitrotoluol und Nitrobenzol erwiesen.

Die erfindungsgemäße Umsetzung wird vorteilhaft bei Temperaturen von 0 bis 70 °C, vorzugsweise 20 bis 50 °C, ausgeführt.

Die erfindungsgemäße Umsetzung der Komponenten (II) und (III) wird vorteilhaft in der Weise vorgenommen, daß man den Katalysator mit dem Lösungsmittel vorlegt und die Reaktionskomponenten einzeln oder als Gemisch zusetzt. In den bei der Verwendung von α,ω-Alkandisäurechloriden als Komponente (II) und/oder Dimethylacrylsäurechlorid bzw. Dimethylacrylsäureanhydrid als Komponente (III) zunächst entstehenden Alkendisäure-α,ω-dichloriden, -α-Chlori-

den-ω-anhydriden oder -α-Ester-ω-chloriden verseifen die Säureanhydrid- bzw. Säurechloridgruppen bereits unter den Bedingungen der Aufarbeitung, so daß nach der Aufarbeitung der Reaktionsmischungen unmittelbar die entsprechenden α,ω-Alkendisäuren bzw. α,ω-Alkendisäuremonoester erhalten werden.

Die erfindungsgemäßen 3-Methyl-5-keto-α,ω-alkendisäuren der Formel (I) fallen als Isomerengemisch an, da die Doppelbindung sowohl in der 2- als auch in der 3-Stellung entsteht. Zur Weiterverarbeitung zu den makrocyclischen β-Methylketonen können diese Isomerengemische unmittelbar, d.h. ohne Trennung in die Isomeren und ohne weitere Reinigung, durch Hydrieren z.B. in alkalischen Medien in Gegenwart üblicher Hydrierungs-Katalysatoren, wie Raney-Nickel, in die entsprechenden 3-Methyl-5-keto-α,ω-alkandisäuren überführt werden.

Die 3-Methyl-5-keto-α,ω-alkandisäuren können anschließend auf verschiedenen Wegen in die makrocyclischen β-Methylketone umgewandelt werden. Zum Beispiel durch Reduktion der Ketogruppe mit Hydrazin nach Wolff-Kishner und Cyclisierung der so erhaltenen 3-Methyl-α,ω-alkandisäuren nach dem von Ziegler et al. (Ann. *504,* (1932), Seite 94) oder dem von Blomquist et al. (J. Am. Chem. Soc. *70,* (1948), Seite 34) beschriebenen Verfahren oder aber — nach Schutz der Ketogruppe — nach dem von Mandapur (Tetrahedron *20,* (1964), Seite 2601) beschriebenen Verfahren durch Acyloinkondensation und anschließende Reduktion des makrocyclischen Acyloins.

Mit den erfindungsgemäßen 3-Methyl-5-keto-α,ω-alkendisäuren der Formel (I) wird eine neue Verbindungsklasse zur Verfügung gestellt, die in einer einzigen Stufe aus leicht zugänglichen Ausgangsverbindungen erhalten wird und mit deren Hilfe 3-Methyl-α,ω-alkandisäuren und α,ω-Alkandisäuren, die eine Ketogruppe in β-Stellung zu einer Methylgruppe enthalten, leicht zugänglich werden. Die genannten α,ω-Alkandisäuren sind wichtige Ausgangsverbindungen für die Herstellung makrocyclischer β-Methylketone.

Makrocyclische β-Methylketone mit 14 bis 16 Ringgliedern, insbesondere das als d,1-Muscon bekannte 3-Methyl-cyclopentadecanon, finden als wichtige Moschus-Riechstoffe und Fixateure in der Parfumerie ausgedehnte Verwendung. Sie können in technischem Maßstab hergestellt werden, indem man 3-Methyl-α,ω-alkandisäuren nach Ziegler (loc. cit.) oder nach Blomquist (loc. cit.) unter Verlust eines Kohlenstoffatoms cyclisiert oder aber α,ω-Alkandisäuren, die eine Ketogruppe in β-Stellung zu einer Methylgruppe enthalten, nach Mandapur (loc. cit.) einer Acyloinkondensation mit anschließender Reduktion unterwirft. Die genannten Verfahren hatten bislang den Nachteil, daß die für ihre Ausführung erforderlichen Alkandisäuren nur durch vielstufige Synthesen erhältlich und daher schwierig zugänglich waren.

Durch die erfindungsgemäßen Verbindungen werden die 3-Methyl-α,ω-alkandisäuren und α,ω-Alkandisäuren, die eine Ketogruppe in β-Stellung zu einer Methylgruppe enthalten, und damit auch die aus ihnen hergestellten makrocyclischen β-Methylketone auf wirtschaftliche Weise herstellbar.

Beispiel 1

Eine Mischung von 276,5 g (1 Mol) α,ω-Dodecandisäuremonoethylesterchlorid und 128 g (1 Mol) β,β-Dimethylacrylsäureethylester wird bei 30 bis 35 °C im Verlauf von 1,5 Stunden zu einer Suspension von 399 g (2,99 Mol) Aluminiumchlorid in 300 ml Methylenchlorid getropft. Nach beendeter Zugabe wird die Reaktionsmischung 3 Stunden auf 45 bis 50 °C erhitzt. Dann wird das Reaktionsgemisch mit Eis hydrolysiert und das Reaktionsprodukt mit Methylenchlorid extrahiert. Nach Abdestillieren des Lösungsmittels werden 370,4 g Rohprodukt erhalten.

Die säulenchromatographische Trennung des Reaktionsproduktes an Kieselgel 60 (Merck) ergibt, daß das Rohprodukt 86 Gew.-% eines Isomerengemisches aus 3-Methyl-5-keto-hexadecen-2-disäurediethylester, 3-Methyl-5-keto-hexadecen-3-disäurediethylester und 3-Methylen-5-keto-hexadecen-disäurediethylester enthält.

Nach mehrmaligem Umkristallisieren des Rohproduktes aus Methanol werden 191,1 g 3-Methyl-5-keto-hexadecen-2-disäurediethylester erhalten.

Fp. : 58-59 °C.

Beispiel 2

Zu einer Suspension von 419 g (3,15 Mol) Aluminiumchlorid und 140 g (0,5 Mol) α,ω-Dodecandisäuredichlorid in 300 ml Methylenchlorid wird bei 30 bis 35 °C ein Gemisch aus 140 g (0,5 Mol) α,ω-Dodecandisäuredichlorid und 134,4 g (1,05 Mol) β,β-Dimethylacrylsäureethylester getropft. Die Reaktionsmischung wird 3,5 Stunden bei 45 bis 50 °C gerührt, dann mit Eiswasser hydrolysiert und mit Methylenchlorid extrahiert. Nach Abziehen des Lösungsmittels verbleiben 376,6 g Rohprodukt.

Das Rohprodukt besteht gemäß säulenchromatographischer Analyse zu 69 Gew.-% aus einem Isomerengemisch aus 3-Methyl-5-keto-hexadecen-2-disäure-1-monoethylester, 3-Methyl-5-keto-hexadecen-3-disäure-1-monoethylester und 3-Methylen-5-keto-hexadecan-disäure-1-monoethylester.

Beispiel 3

276,5 g (1 Mol) α,ω-Dodecandisäuremonoethylesterchlorid und 156 g (1 Mol) β,β-Dimethylacrylsäure-n-butylester werden wie in Beispiel 1 beschrieben umgesetzt. Es werden 405 g Rohprodukt erhalten.

Die Auftrennung des Rohproduktes mittels Säulenchromatographie an Kieselgel 60 (Merck) ergibt, daß dieses 82 Gew.-% einer Isomerenmischung aus 3-Methyl-5-keto-hexadecen-2-disäu-

re-α-n-butyl-ω-ethylester, 3-Methyl-5-keto-hexadecen-3-disäure-α-n-butyl-ω-ethylester und 3-Methylen-5-keto-hexadecan-disäure-α-n-butyl-ω-ethylester enthält.

Beispiel 4

130 g (0,5 Mol) α,ω-Undecandisäure-monoethylesterchlorid und 64 g (0,5 Mol) β,β-Dimethylacrylsäureethylester werden wie im Beispiel 1 beschrieben umgesetzt. Es Werden 170 g Rohprodukt erhalten.

Die Auftrennung des Rohproduktes mittels Säulenchromatographie an Kieselgel 60 (Merck) ergibt, daß das Rohprodukt 84 Gew.-% einer Isomerenmischung aus 3-Methyl-5-keto-pentadecen-2-disäure-diethylester, 3-Methyl-5-keto-pentadecen-3-disäure-diethylester und 3-Methylen-5-keto-pentadecan-disäure-diethylester enthält.

Beispiel 5

a) 150 g (0,41 Mol) 3-Methyl-5-keto-hexadecen-2-α,ω-disäurediethylester (Fp.: 58-59 °C, hergestellt nach Beispiel 1) werden mit einer Lösung von 50 g (0,9 Mol) Kaliumhydroxid in 240 ml Wasser und 500 ml Ethanol versetzt und nach Zugabe von 16 g Raney-Nickel bei 40 °C und 40 Atm Wasserstoffdruck hydriert. Die Wasserstoffaufnahme beträgt 100 % der Theorie.

Nach Abfiltrieren des Katalysators wird die Lösung eingeengt, mit Salzsäure angesäuert und das Reaktionsprodukt mit Essigester extrahiert.

Es werden 125,5 g (98 % der Theorie) 3-Methyl-5-ketohexadecan-disäure erhalten.

Fp.: 84-85 °C.

b) 35 g (0,11 Mol) 3-Methyl-5-keto-hexadecandisäure (wie vorstehend unter a) beschrieben erhalten) werden mit 30,8 g (0,55 Mol) Kaliumhydroxid, 150 ml Diethylenglykol und 20,6 g (0,33 Mol) 80 %igem Hydrazinhydrat 2 Stunden auf Rückflußtemperatur erhitzt. Dann wird Wasser und überschüssiges Hydrazin abdestilliert und eine Stunde auf 220 °C erhitzt. Nach dem Abkühlen auf 15 °C wird die Reaktionsmischung mit Salzsäure angesäuert, und mit Essigester extrahiert. Der nach dem Entfernen des Essigesters aus der organischen Phase verbleibende Rückstand wird aus Petrolether umkristallisiert. Es werden 30,3 g (91 % der Theorie) 3-Methyl-hexadecandisäure erhalten.

Fp.: 77-78 °C.

Die Ausbeute an 3-Methyl-hexadecandisäure bezogen auf 3-Methyl-5-keto-hexadecen-2-α,ω-disäurediethylester beträgt 89 % der Theorie.

Wird anstelle des kristallinen 3-Methyl-5-keto-hexadecen-2-disäurediethylesters das nach dem Abdestillieren des Lösungsmittels anfallende Rohprodukt des Beispiels 1 in die vorstehend beschriebenen Reduktionen a) und b) eingesetzt, so wird die 3-Methyl-hexadecandisäure in einer Ausbeute von 62 % der Theorie, bezogen auf eingesetztes Rohprodukt, erhalten.

Beispiel 6

a) 150 g (0,41 Mol) 3-Methyl-5-keto-hexadecen-2-disäurediethylester (Fp.: 58-59 °C erhalten nach Beispiel 1) werden in 1 000 ml Methanol gelöst und nach Zusatz von 16 g Raney-Nickel bei 20 °C und 40 Atm Wasserstoffdruck hydriert. Die Wasserstoffaufnahme beträgt praktisch 100 % der Theorie. Nach dem Abfiltrieren des Katalysators wird das Lösungsmittel abgezogen.

Es werden 148 g 3-Methyl-5-keto-hexadecan-α,ω-disäurediethylester erhalten.

Fp.: 25 °C.

b) 148 g (0,4 Mol) 3-Methyl-5-keto-hexadecan-α,ω-dicarbonsäurediethylester (wie vorstehend unter a) beschrieben erhalten) werden mit 2 Mol Kaliumhydroxid und 1,2 Mol Hydrazinhydrat wie in Beispiel 5 b) beschrieben reduziert.

Es werden 108 g (= 90% der Theorie) 3-Methylhexadecandisäure erhalten.

Fp.: 77 °C.

Die Ausbeute an 3-Methyl-hexadecandisäure bezogen auf 3-Methyl-5-keto-hexadecen-2-disäureethylester beträgt 90 % der Theorie.

Beispiel 7

300 g des nach Beispiel 2 erhaltenen Rohproduktes werden unter den in Beispiel 5 a) beschriebenen Bedingungen hydriert und anschließend ohne weitere Reinigung unter den in Beispiel 5 b) beschriebenen Bedingungen reduziert. Es werden 140 g (56 % der Theorie, bezogen auf eingesetztes Rohprodukt) 3-Methyl-hexadecan-α,ω-disäure erhalten.

**Ansprüche**

1. 3-Methyl-5-keto-α,ω-alken-disäuren und deren Ester der allgemeinen Formel

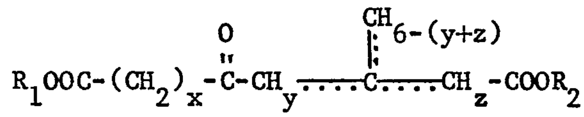

in der

sich die Doppelbindung in einer durch die gestrichelten Linien gezeigten Stellungen befindet,

$R_1$ und $R_2$ unabhängig voneinander für ein Wasserstoffatom oder eine $C_1$-$C_4$-Alkylgruppe stehen,

x eine ganze Zahl von 9 bis 11 bedeutet und

y und z unabhängig voneinander für 1 oder 2 stehen, mit der Maßgabe, daß y und z nicht gleichzeitig 1 sein dürfen.

2. Verbindungen der gemäß in Anspruch 1 angegebenen Formel, in der

$R_1$ und $R_2$ unabhängig voneinander für Wasserstoff, eine Methyl- oder Ethylgruppe stehen.

3. Verfahren zur Herstellung von 3-Methyl-5-keto-α,ω-alkendisäuren und deren Ester der Formel

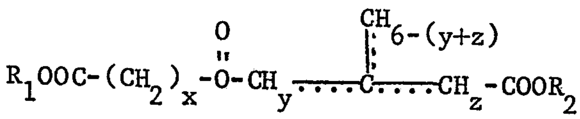

in der

sich die Doppelbindung in einer durch die gestrichelten Linien gezeigten Stellungen befindet,

$R_1$ und $R_2$ unabhängig voneinander für ein Wasserstoffatom oder eine $C_1$-$C_4$-Alkylgruppe stehen,

x eine ganze Zahl von 9 bis 11 bedeutet und

y und z unabhängig voneinander für 1 oder 2 stehen, mit der Maßgabe, daß y und z nicht gleichzeitig 1 sein dürfen,

dadurch gekennzeichnet, daß man ein α-ω-Alkandisäure-derivat der allgemeinen Formel

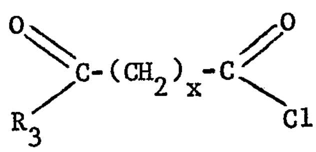

in der

x die vorstehend angegebene Bedeutung aufweist und

$R_3$ für Chlor oder eine $C_1$-$C_4$-Alkoxygruppe steht,

mit einem Dimethylacrylsäure-Derivat der allgemeinen Formel

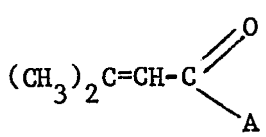

in der

A für Chlor, einen $C_1$-$C_4$-Alkoxy- oder den β,β-Dimethylacryloyloxirest steht,

in Gegenwart einer Lewis-Säure umsetzt.

4. Verwendung der 3-Methyl-5-keto-α,ω-alkendisäuren und deren Ester gemäß Anspruch 1 und 2 zur Herstellung makrocyclischer β-Methyl-ketone.

**Claims**

1. 3-Methyl-5-keto-α,ω-alkenedioic acids and their esters of the general formula

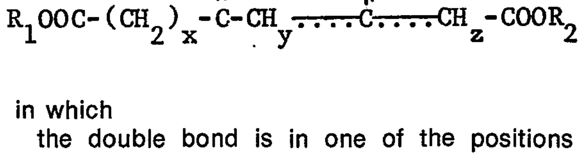

in which

the double bond is in one of the positions indicated by the broken lines,

$R_1$ and $R_2$ independently of one another represent a hydrogen atom or a $C_1$-$C_4$-alkyl group,

x denotes an integer from 9 to 11 and

y and z independently of one another are 1 or 2, with the proviso that y and z may not be 1 at the same time.

2. Compounds of the formula given in Claim 1 in which

$R_1$ and $R_2$ independently of one another are hydrogen, or a methyl or ethyl group.

3. Process for the preparation of 3-methyl-5-keto-α,ω-alkenedioic acids and their esters of the formula

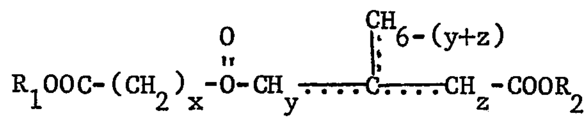

in which

the double bond is in one of the positions indicated by the broken lines,

$R_1$ and $R_2$ independently of one another represent a hydrogen atom or a $C_1$-$C_4$-alkyl group,

x denotes an integer from 9 to 11 and

y and z independently of one another are 1 or 2, with the proviso that y and z may not be 1 at the same time.

characterised in that an α,ω-alkanedioic acid derivative of the general formula

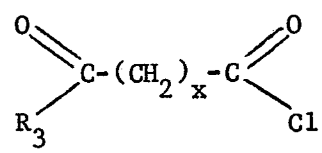

in which

x has the meaning given above and

$R_3$ represents chlorine or a $C_1$-$C_4$-alkoxy group is reacted with a dimethylacrylic acid derivative of the general formula

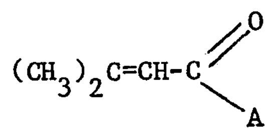

in which

A represents chlorine, a $C_1$-$C_4$-alkoxy radical or the β,β-dimethylacryloyloxy radical in the presence of a Lewis acid.

4. Use of the 3-methyl-5-keto-α,ω-alkenedioic acids and their esters according to Claims 1 and 2 for the preparation of macrocyclic β-methyl-ketones.

**Revendications**

1. Acides 3-méthyl-5-céto-α,ω-alcène-dioïques et leurs esters de formule générale

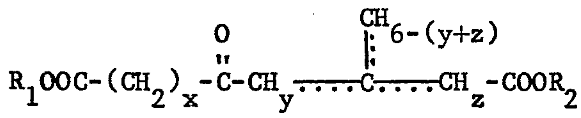

dans laquelle

la double liaison se trouve dans l'une des positions indiquées par les traits en pointillé,

$R_1$ et $R_2$ représentent chacun, indépendamment l'un de l'autre, un atome d'hydrogène ou un groupe alkyle en $C_1$-$C_4$,

x est un nombre entier de 9 à 11 et

y et z représentent chacun, indépendamment l'un de l'autre, 1 ou 2, avec la restriction que y et z ne peuvent représenter tous deux 1.

2. Composés de formule indiquée dans la revendication 1, dans laquelle

$R_1$ et $R_2$ représentent chacun, indépendamment l'un de l'autre, l'hydrogène, un groupe méthyle ou éthyle.

3. Procédé de préparation des acides 3-méthyl-5-céto-α,ω-alcène-dioïques et de leurs esters de formule

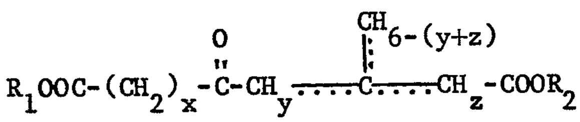

dans laquelle

la double liaison se trouve dans l'une des positions indiquées par les traits en pointillé,

$R_1$ et $R_2$ représentent chacun, indépendamment l'un de l'autre, un atome d'hydrogène ou un groupe alkyle en $C_1$-$C_4$,

x est un nombre entier de 9 à 11 et

y et z représentent chacun, indépendamment l'un de l'autre, 1 ou 2, avec la restriction que y et z ne peuvent représenter tous deux 1, caractérisé en ce que l'on fait réagir un dérivé d'acide, α-ω-alcane-dioïque de formule générale

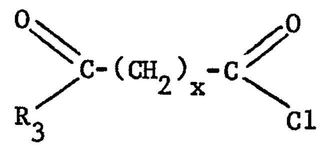

dans laquelle

x a les significations indiquées ci-dessus et

$R_3$ représente le chlore ou un groupe alcoxy en $C_1$-$C_4$, avec un dérivé de l'acide diméthylacrylique de formule générale

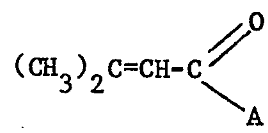

dans laquelle

A représente le chlore, un groupe alcoxy en $C_1$-$C_4$ ou le reste β,β-diméthylacryloyloxy, en présence d'un acide de Lewis.

4. Utilisation des acides 3-méthyl-5-céto-α,ω-alcène-dioïques et leurs esters selon les revendications 1 et 2 pour la préparation de β-méthylcétones macrocycliques.